(19) Europäisches Patentamt
European Patent Office
Office européen des brevets

(11) Publication number: 0 443 518 A2

# (12) EUROPEAN PATENT APPLICATION

(21) Application number: **91102346.3**

(22) Date of filing: **19.02.91**

(51) Int. Cl.[5]: **A61K 31/70**, G01N 33/574, A61K 39/00

(30) Priority: **19.02.90 JP 39044/90**

(43) Date of publication of application: **28.08.91 Bulletin 91/35**

(84) Designated Contracting States: **AT BE CH DE DK ES FR GB GR IT LI LU NL SE**

(71) Applicant: **MECT CORPORATION**
**2-1-1, Nishi-Shinjyuku Shinjyuku-ku**
**Tokyo(JP)**

(72) Inventor: **Nagai, Yoshitaka**
**1-29-10, Akatsutsumi**
**Setagaya-ku, Tokyo(JP)**
Inventor: **Suda, Isao**
**Takado-so, 2886-5, Yayoi-cho**
**Tokorozawa-shi, Saitama-ken(JP)**
Inventor: **Hino, Akihiro**
**Kojima-haimu 2-103, 2-628-5, Sayamagaoka**
**Tokorozawa-shi, Saitama-ken(JP)**
Inventor: **Sugimoto, Mamoru**
**1-26-8, Kamitakada**
**Nakano-ku, Tokyo(JP)**
Inventor: **Tanaka, Makoto**
**760-6, Shimomakuri**
**Koshigaya-shi, Saitama-ken(JP)**
Inventor: **Ito, Masayoshi**
**1-27-22-303, Fujimidai**
**Kunitaci-shi, Tokyo(JP)**
Inventor: **Ogawa, Tomoya**
**3-6-6-3-101, Kita-machi, Kichijyoji**
**Musashino-shi, Tokyo(JP)**

(74) Representative: **Vossius & Partner**
**Siebertstrasse 4 P.O. Box 86 07 67**
**W-8000 München 86(DE)**

## (54) Pharmaceutical containing episialo complex carbohydrate.

(57) A drug for diagnosis using a serum sample, particularly a drug for diagnosis using a serum sample for the detection of cancer containing an episialo complex carbohydrate such as $epiGM_3$, $epiGM_4$ and $epiGM_5$, a cancer vaccine containing the episialo complex carbohydrate, a method for diagnosis using a serum sample, particularly a diagnostic method for the prevention and therapy of cancer are disclosed.

EP 0 443 518 A2

This invention relates to a drug for diagnosis using a serum sample using an episialo complex carbohydrate, more particularly a drug for the diagnosis of cancer using a serum sample. This invention also relates to a cancer vaccine, and to a method for diagnosis using a serum sample, more particularly a method for the prevention and therapy of cancer.

A carbohydrate chain contributes an important part to the antigenicity on the surface of cells and carbohydrate chain antigens on the surface of cells may become abnormal and change to cancer antigens. Recently, the possibility of therapy and serum diagnosis of cancer using monoclonal antibodies to a cancer antigen has been strongly suggested.

Further, it is possible to diagnose the presence of cancer in a body by examining the existence of an antibody to a cancer antigen. It is therefore necessary to provide an antibody reactive with a cancer antigen.

An object of this invention is to provide an antibody reactive with a cancer antigen.

Another object of this invention is to provide a drug for the diagnosis and preventive treatment of cancer and methods using such an antibody.

The present inventors discovered an antibody recognizing an episialo complex carbohydrate in an animal serum.

This invention relates to a drug for diagnosis using a serum sample containing an episialo complex carbohydrate, more particularly a drug for diagnosis of cancer using a serum sample, and to an in vitro method for diagnosis using a serum sample comprising reacting an episialo complex carbohydrate with a serum sample, more particularly a diagnostic in vitro method for the detection of cancer using a serum sample.

Another embodiment of this invention is a cancer vaccine containing an episialo complex carbohydrate.

Fig. 1 illustrates the reactivity of a serum of a normal mouse with $GM_3$ and $epiGM_3$.

Fig. 2 illustrates the reactivity of a serum of a mouse implanted with a tumor with $GM_3$ and $epiGM_3$.

Fig. 3 illustrates the reactivity of a serum of a mouse implanted with a tumor with $GM_3$, $epiGM_3$, $GM_4$, $epiGM_4$, $GM_5$ and $epiGM_5$.

Fig. 4 illustrates the inhibitory effect of N-acetylneuraminic acid to the reactivity of a serum of a mouse implanted with a tumor with $epiGM_3$.

This invention will be explained in more detail below.

Gangliosides are a kind of complex carbohydrates. The binding configuration of acetylneuraminic acid existing in natural gangliosides is of the $\alpha$ type. However, recently, so-called episialo complex carbohydrates in which the binding configuration of acetylneuraminic acid is of the $\beta$ type have been synthesized through a chemical synthesis of gangliosides.

All conventional carbohydrate chain markers have the $\alpha$ type binding configuration of acetylneuraminic acid and it was not known that episialo complex carbohydrates can function as a carbohydrate chain cancer marker.

The incompletion (i.e. generation of abnormal carbohydrate) of carbohydrate presently known results from the abnormality of patterns of carbohydrate combinations in carbohydrate chains synthesized in cancer cells. Further, it has recently been reported that upon generation of cancer N-glycolyl neuraminic acid (NeuGc) appears. Sialic acids of humans are of the N-acetyl type except for the fetal period. N-glycolyl neuraminic acid is easily obtained by changing the N-acetyl group at the 5-position of acetylneuraminic acid to a hydroxyl group. That is, the incompletion of carbohydrate presently known is based on a difference of substituents in a carbohydrate chain or a difference of the pattern of carbohydrate combinations.

That is, it was not known that a material having a carbohydrate chain abnormality resulting from the configuration type, such as episialo complex carbohydrates utilized in this invention appears upon the generation of a tumor.

This invention is based on the knowledge that the amount of an antibody recognizing an episialo complex carbohydrate is increased in a serum obtained from a tumor bearing animal. This suggests that episialo complex carbohydrates which have not been discovered in normal cells exist in a tumor bearing animal.

Episialo complex carbohydrates of this invention are gangliosides in which the binding configuration of acetylneuraminic acid is of the $\beta$ type. Examples of episialo complex carbohydrates include $epiGM_1$, $epiGM_2$, $epiGM_3$, $epiGM_4$ and $epiGM_5$.

$EpiGM_3$ is a compound (28) described in JP-B-1-49354 and in US-A-4,968,786, and is prepared according to the method described in these documents. Chemical structures of $GM_3$ and $epiGM_3$ are as follows:

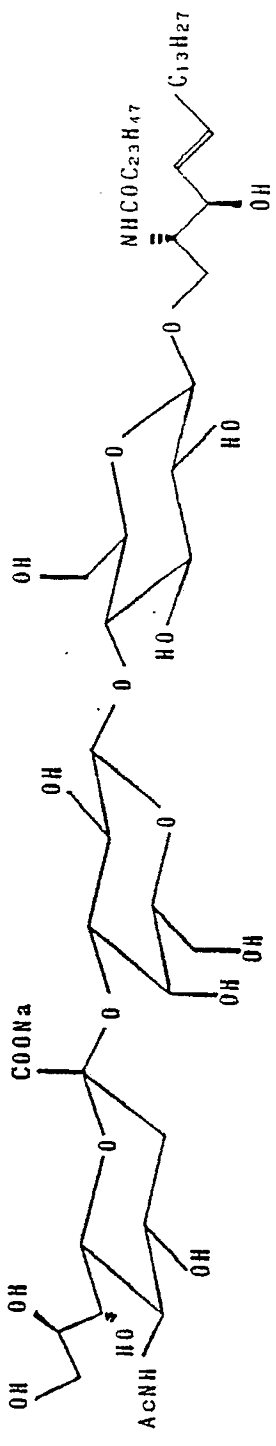

GM$_3$

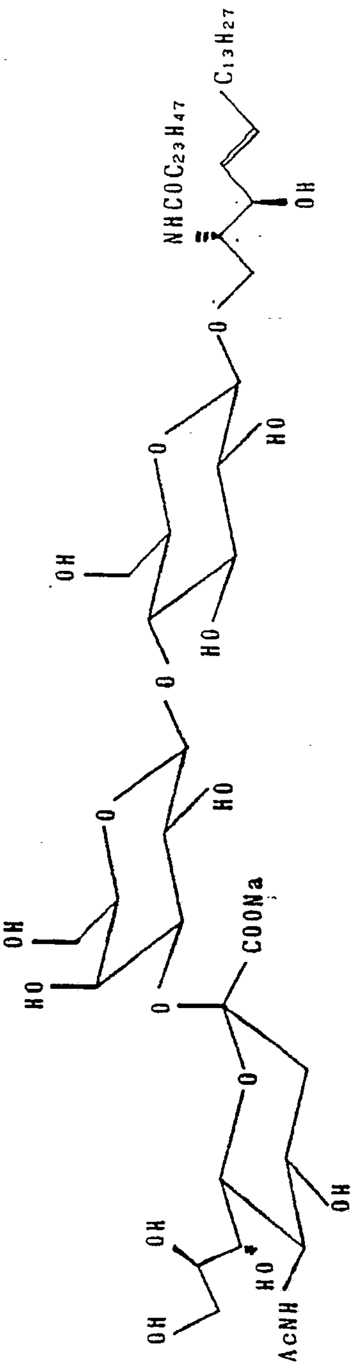

epiGM$_3$

EpiGM$_4$ is a compound (27) described in JP-A-61-282391 and in US-A-4,751,290, and is prepared according to the method described in these documents. Chemical structures of GM$_4$ and epiGM$_4$ are as follows:

# GM₄

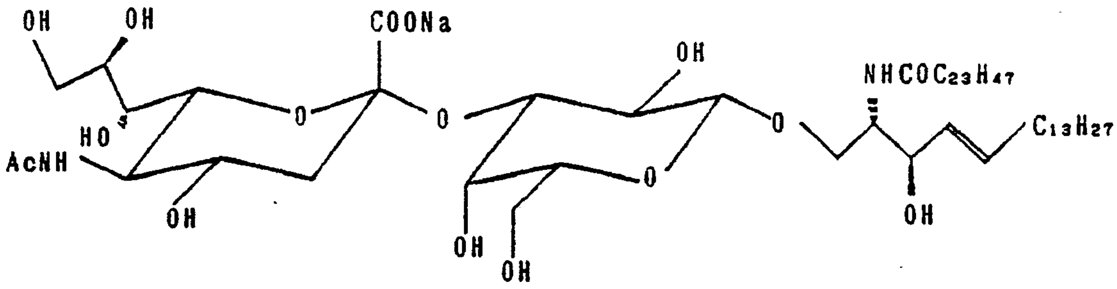

# epiGM₄

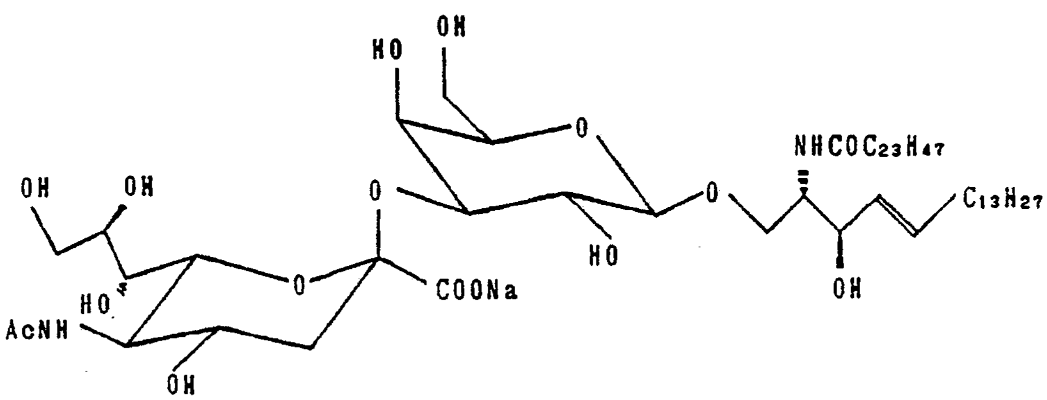

$EpiGM_5$ is a compound (III) described in JP-A-63-452931 and in US-A-4,730,058, and is prepared according to the method described in these documents. Chemical structures of $GM_5$ and $epiGM_5$ are as follows:

GM5

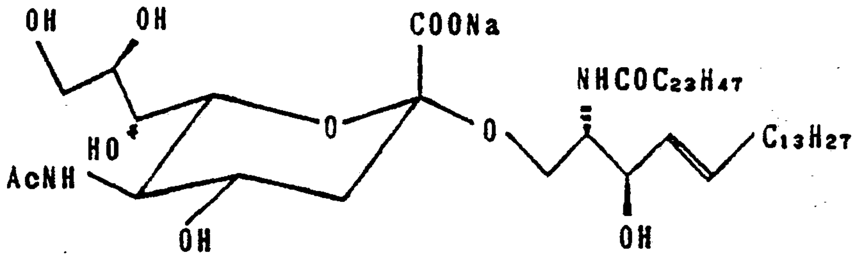

epi GM5

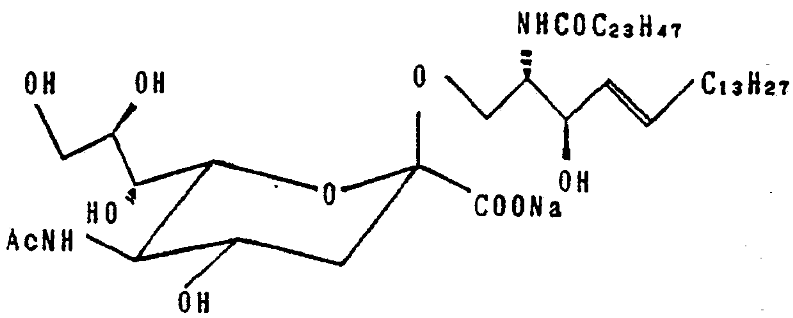

In accordance with this invention, it is possible to conduct a diagnosis using a serum sample, particularly a diagnosis of cancer using a serum sample and using episialo complex carbohydrates.

A diagnosis using a serum sample and using episialo complex carbohydrates can be carried out in the same manner as that used in conventional serum diagnosis. That is, an episialo complex carbohydrate is incubated with an animal serum and then the existence of antibody which reacted with the episialo complex carbohydrate and, if necessary, the amount of the antibody is examined using a generally known examining method such as ELISA or RIA.

It is preferred to use a drug for diagnosis by dissolving an episialo complex carbohydrate in a solvent such as ethanol or methanol. The concentration of the episialo complex carbohydrate in the solvent suitably ranges, for example, between 1 and 1000 ng/ml.

According to the method for diagnosis using a serum sample of this invention, it is possible to diagnose cancer, infection diseases, genetic diseases and immunological diseases.

Further, this invention provides a cancer vaccine containing (an) episialo complex carbohydrate (s).

This cancer vaccine is the episialo complex carbohydrate(s) per se or a mixture of the episialo complex carbohydrate(s) and an adjuvant such as liposomes of BCG (Bacille de Calmette et Guérin) or the like.

The cancer vaccine of this invention is preferably administered 4 to 8 times within 1 to 4 weeks, and 1 to 1000 μg of the episialo complex carbohydrate is preferably administered per dose.

According to the method for diagnosis using the serum sample and using the episialo complex carbohydrate, it is possible to diagnose cancer, infection diseases, genetic diseases and immunological diseases.

It is possible to provide immunity against cancer by using the cancer vaccine of this invention.

## EXAMPLE

This invention will be illustrated by the following examples.

### Gangliosides

Episialo complex carbohydrate $GM_3$ and ganglioside $GM_3$ were synthesized in accordance with the method described in the Japanese patent publication No. 1-49354. Episialo complex carbohydrate $GM_4$ and ganglioside $GM_4$ were synthesized in accordance with the method described in the Japanese patent disclosure No. 61-282391. Episialo complex carbohydrate $GM_5$ and ganglioside $GM_5$ were synthesized in accordance with the method described in Japanese patent disclosure 63-45293.

### Mouse

Mice of various inbred lines bought from Charles River Japan Co. Ltd. and Japan SLC Co. Ltd. and kept under the SPF conditions were used.

### Serum

A serum was obtained by clotting of blood which was collected from a mouse retroorbitally (eyeground method) and removing the clotted blood by centrifugation.

### ELISA Assay

ELISA assay was conducted by the conventional method.

100 ng of each ganglioside dissolved in ethanol was added to each well of a 96-well microtiter plate and then allowed to stand at 37°C to remove ethanol completely. A blocking solution comprising a phosphate buffer solution (PBS) containing 5% of bovine serum albumin (BSA) or the like was added to the wells and the plate was incubated at 37°C for 2 hours. After the blocking solution was removed, the collected mouse serum was added to each well and the plate was incubated at 37°C for 1 hour. Each well was washed with a phosphate buffer solution containing 1% of BSA. Anti-mouse IgG, IgM and IgA antibodies which were labeled with peroxidase were added to each well and the plate was incubated at 37°C for 1 hour. Each well was washed with a phosphate buffer solution containing 1% of BSA. A substrate solution containing 400 μg/ml of o-phenylenediamine and 0.001% of hydrogen peroxide was added to each well, and the reaction was conducted at room temperature for 15 minutes. 8N sulfuric acid was added to stop the reaction and then the absorbance of the reaction mixture in each well was measured at 490 nm by using a spectrophotometer for a microtiter plate.

### Results

(1) Reactivity of mouse serum with epi$GM_3$

As shown in Fig. 1, normal sera derived from C57BL/6 femal mice (9 weeks old) were reacted with epi$GM_3$ (shown by a solid line). However, the normal sera derived from C57BL/6 female mice did not react with $GM_3$ (shown by a broken line).

As shown in Fig. 2, sera derived from C57BL/6 female mice (9 weeks old) implanted with B16 melanoma cells (2 weeks after implantation) also reacted with epi$GM_3$ (shown by a solid line). The reaction level was remarkably increased. However, these sera did not react with $GM_3$ (shown by a broken line). In Fig. 2, ●, △, ▲ and □ show the data with respect to a serum derived from a tumor the diameter of which is

5 mm or less, 6 mm, 8 mm and 16 mm, respectively.

By comparing the results of Fig. 1 with those of Fig. 2, it will be clear that the reactivity with epi$GM_3$ of a serum derived from the mice implanted with tumor cells is stronger than that of sera derived from normal mice.

Values shown in Fig. 1 and Fig. 2 were obtained by subtracting background values and taking the average of three wells.

(2) Reactivity of mouse serum with gangliosides other than epi$GM_3$

The serum which was derived from the mouse with a 6 mm tumor and which shows the strongest reactivity among the sera derived from C57BL/6 female mice (9 weeks old) implanted with B16 melanoma cells as described above (1) was diluted 200 times and the reactivity with $GM_3$, epi$GM_3$, $GM_4$, epi$GM_4$, $GM_5$ and epi$GM_5$ was tested. As shown in Fig. 3, the reactivity with epi$GM_3$ was the strongest and the reactivity with epi$GM_4$ was the second strongest.

Values shown in Fig. 3 were obtained by subtracting background values and taking the average of three wells.

(3) Inhibition of the reactivity of mouse serum with epi$GM_3$ by acetylneuraminic acid

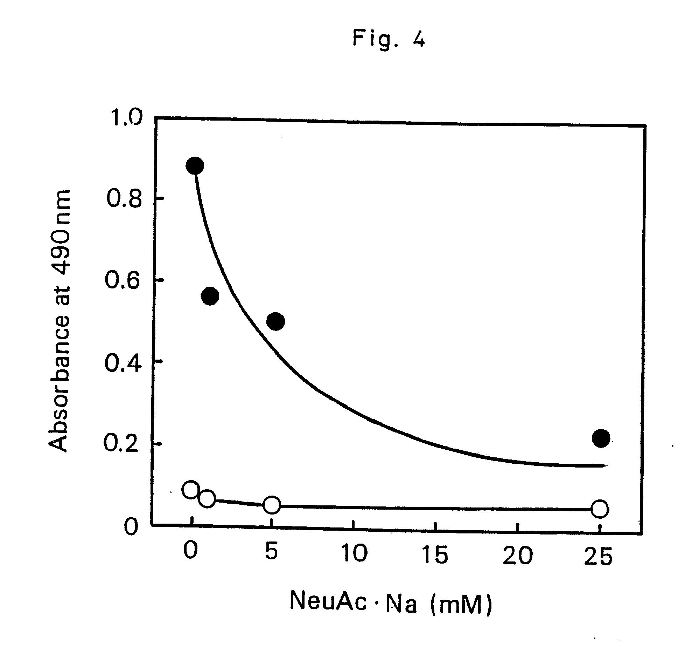

Sodium N-acetylneuraminate solutions of various concentration were added to the same sera of mice as those used in the above (2). The reactivity with epi$GM_3$ (shown by filled circles) and with $GM_3$ (shown by open circles) were tested and are shown in Fig. 4. Values shown in Fig. 4 were obtained by subtracting background values and taking the average of three wells. In order to avoid the effect of pH reduction in the reaction solution, the sodium salt of acetylneuraminic acid was used.

As can be seen from the results in Fig. 4, the reactivity of mouse serum with epi$GM_3$ was inhibited by N-acetylneuraminic acid whose configuration of a hydroxyl group is of the $\beta$ type. This strongly suggests that the strong reactivity of the mouse serum with epi$GM_3$ results from the $\beta$ configuration of the acetylneuraminic acid group in epi$GM_3$.

## Claims

1. A drug for diagnosis using a serum sample containing at least one episialo complex carbohydrate.

2. The drug for diagnosis using a serum sample of claim 1 wherein the episialo complex carbohydrate is selected from epi$GM_3$, epi$GM_4$ and epi$GM_5$.

3. A drug for diagnosis using a serum sample for the detection of cancer containing at least one episialo complex carbohydrate.

4. The drug of claim 3 wherein the episialo complex carbohydrate is selected from epi$GM_3$, epi$GM_4$ and epi$GM_5$.

5. A cancer vaccine containing at least one episialo complex carbohydrate.

6. The cancer vaccine of claim 5 wherein the episialo complex carbohydrate is selected from epi$GM_3$, epi$GM_4$ and epi$GM_5$.

7. A method for diagnosis using a serum sample comprising reacting a serum with an episialo complex carbohydrate.

8. The method of claim 7 wherein the episialo complex carbohydrate is selected from epi$GM_3$, epi$GM_4$ and epi$GM_5$.

9. A method for diagnosis of cancer comprising reacting a serum with an episialo complex carbohydrate.

10. The method of claim 9 wherein the episialo complex carbohydrate is selected from epi$GM_3$, epi$GM_4$ and epi$GM_5$.

Fig. 1

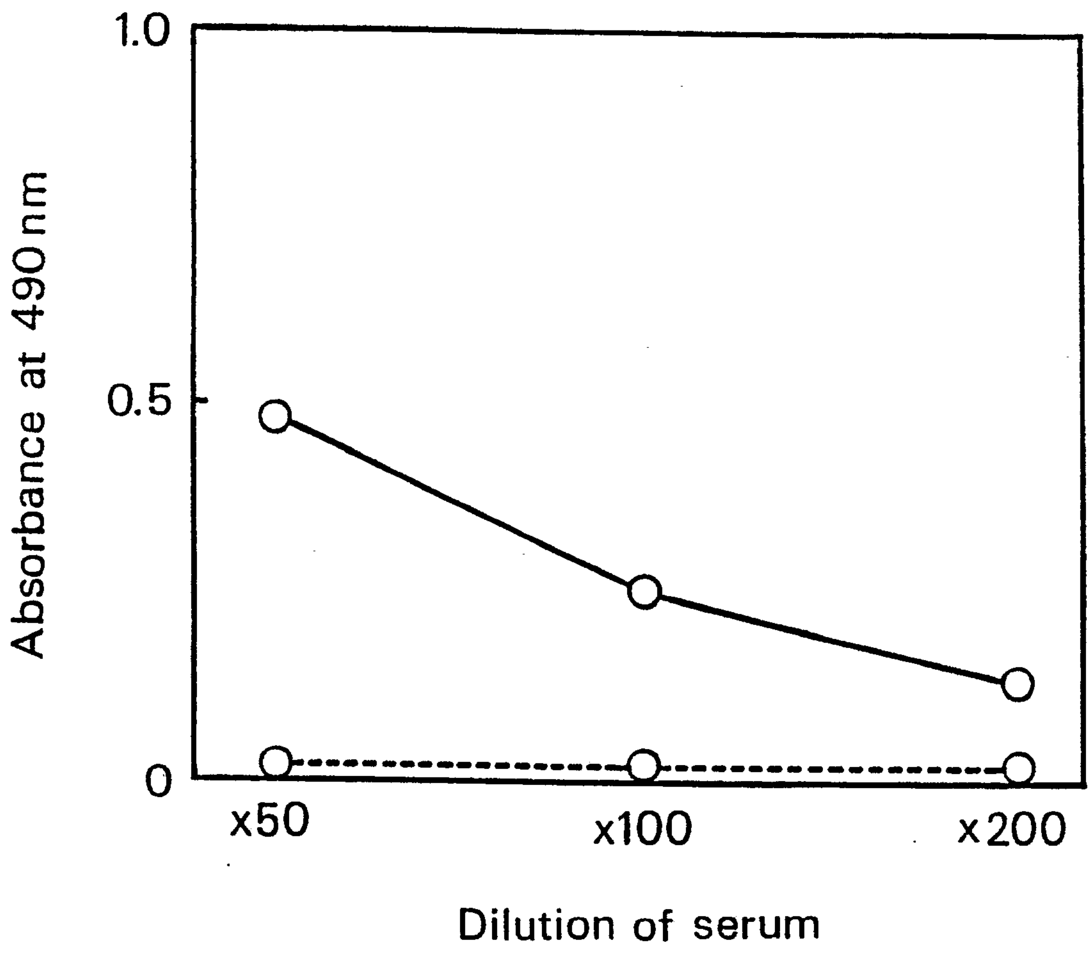

F i g. 2

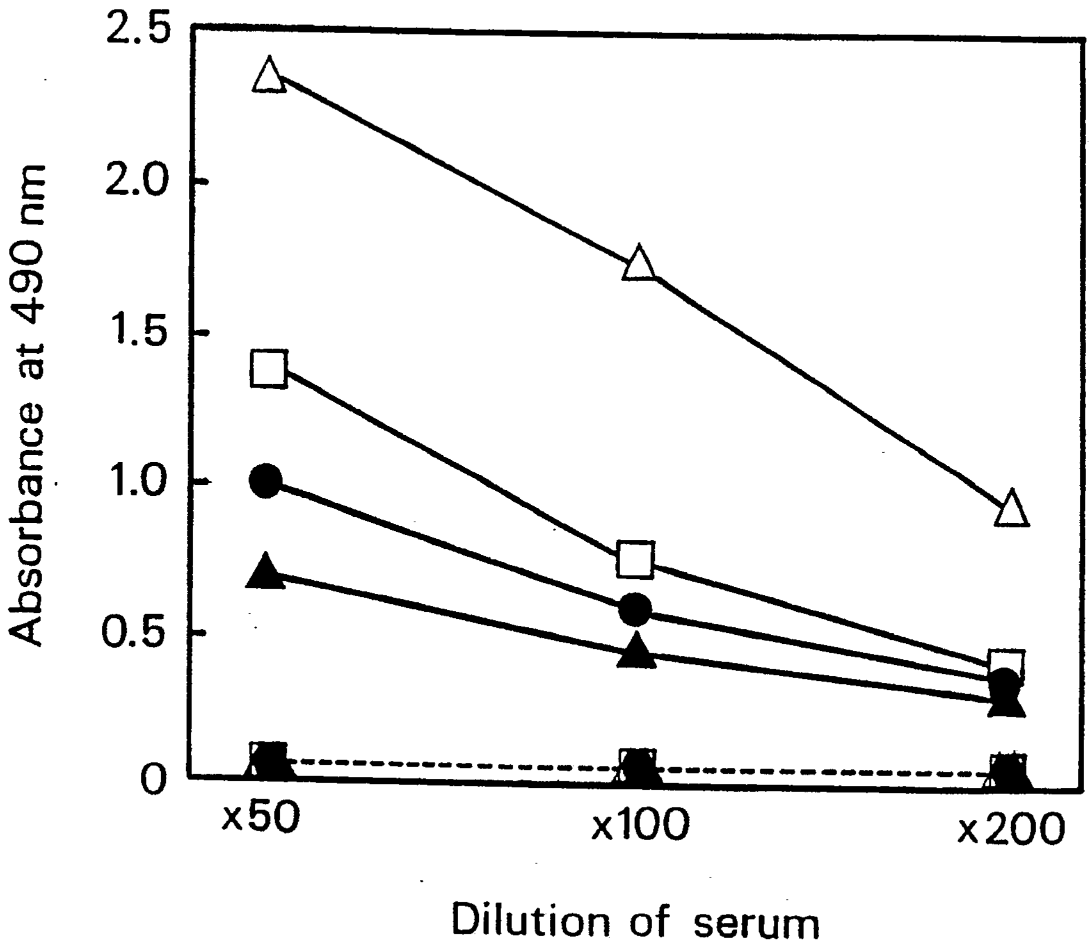
2.5
2.0
1.5
1.0
0.5
0
Absorbance at 490 nm
x50
x100
x200
Dilution of serum

Fig. 3

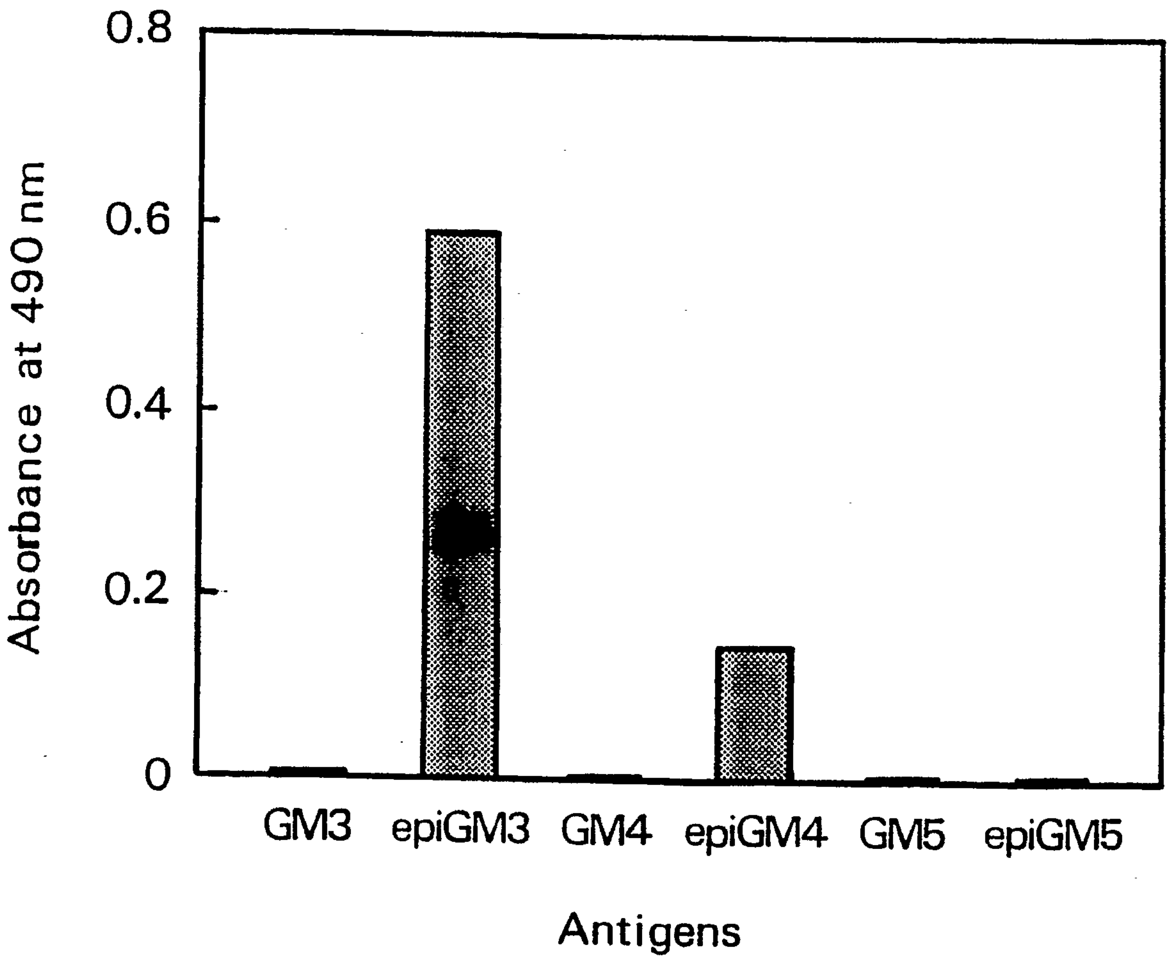
0.8
0.6
0.4
0.2
0
Absorbance at 490 nm
GM3
epiGM3
GM4
epiGM4
GM5
epiGM5
Antigens

Fig. 4